# EUROPEAN PATENT APPLICATION

(11) **EP 2 246 659 A1**
(43) Date of publication of application: **03.11.2010**
(21) Application number: 09158892.1
(22) Date of filing: 28.04.2009
(51) Int. Cl.: G01B 9/02

(54) **Optical coherence tomography apparatus and method**

(71) Applicant: Optopol Technology S.A., 42-400 Zawiercie (PL)
(72) Inventor: Bajraszewski, Tomasz, 87-100 Torun (PL); Szkulmowski, Maciej, 87-100 Torun (PL)
(74) Representative: Glawe, Delfs, Moll

(57) **Abstract**

The invention relates to optical coherence tomography using fiber optics. The light intensity in the reference arm is controlled in a rapid manner by an intentional misalignment of the reference light.

## Description

The invention relates to an apparatus for and method of Fourier domain optical coherence tomography.

Optical coherence tomography (OCT) is a technique for examination of three-dimensional structures of partially transparent matter. According to this technique, low coherent light is divided into two portions. One portion is used as sample light to be passed into a sample arm to illuminate the sample under investigation. The second portion is used as reference light and led through a reference arm to be recombined at a point of recombination with the light back-scattered from the sample. The recombined light contains an interferometric signal carrying information about the internal structure of the sample. This information can be retrieved in essentially two different ways.

The first way, known as "Time domain Optical Coherence Tomography" (TdOCT), is based on a scannable optical path delay introduced in the reference arm. The delay is scanned in an oscillating manner. In this case, interference fringes occur only in certain scan positions, namely in positions in which the optical path length of the reference path matches the optical path length for the light back-scattered by the sample. This facilitates determination of the relative distances of back-scattering structures within the sample (see Huang et al., Science, Vol. 254, 1991, p. 1178 to 1181).

The second way for retrieving information from the recombined light, known as "Fourier domain Optical Coherence Tomography" (FdOCT), is based on spectral analysis of the recombined light. The spectrum of the recombined light, i.e. the distribution of the light intensities for the various spectral components, is recorded. This can be performed by using either a spectrometer ("Spectral Optical Coherence Tomography", SOCT; see Szkulmowska et al., Journal of Physics D: Applied Physics, Vo. 38, 2005, 2606 - 2611), or a tuned light source ("Swept Source Optical Coherence Tomography", SS-OCT; see R. Huber et al., Optics Express, Vol. 13, 2005, 3513 - 3528).

Common OCT devices comprise means for scanning the light beam illuminating the eye transversally, i.e. in one or two directions perpendicular to the axis of said light beam. A two- or three-dimensional image of the internal sample structure is thus generated. The axis parallel to the light beam is usually called the z axis and the recordal of the information about the structure along this axis is called "A scan". The other perpendicular axes are called x and y axes. A set of A scans taken along the x axis forms a two-dimensional "xz" tomogram called "B scan", while a set of B scans acquired along the y axis forms a volume data set representing the three-dimensional structure under investigation.

FdOCT is nowadays regarded as superior over TdOCT due to its significantly better signal-to-noise ratio (SNR) (US 2007/0115476 A1, paragraph [0009]). However, even FdOCT suffers from poor SNR if the sample light is attenuated significantly. This is, for instance, a problem for ophthalmic OCT devices when patients with cataract are examined. It is possible to maintain a good SNR in such situations by increasing the energy of the sample light used for the detection, in particular by extending the detection time. However, as the detectors in FdOCT systems are usually operated close to their saturation level this level could be exceeded which would spoil the entire measurement.

The object of the invention is to provide a countermeasure which allows for keeping the operation below saturation level even if the energy of the sample light used for the detection is increased. This is achieved by the subject-matter defined in claims 1 and 7. The sub-claims define further improvements of the invention.

The invention compensates for the increment of sample light energy by reducing the energy of the reference light. This is achieved by an intentional misalignment in the reference arm which causes the intensity of the light returning from the reference arm to drop. A major advantage of using a misalignment is that it allows for rapid attenuation changes during the measurement.

A light source according to the invention can be any device emitting electromagnetic radiation within a vacuum wavelength range from 600 nm to 1,700 nm. A plurality of light sources can be combined to form a light source according to the invention, in particular to extend the spectral range (cf. Cense et al., Optics Express 2004, 2435 ff.; WO 2007/016296, paragraph [0017]).

The reference arm and the sample arm can be designed in various ways as will be known by the person skilled in the field of interferometry. The invention may be used for various interferometer types including Michelson types and Mach-Zehnder types (e.g. US 2007/0291277 Al, Fig. 1). Likewise, the recombining device can by of any type known in the field of interferometry. Optical couplers are preferred as recombining devices.

The detection device is, in case of SOCT, a spectrometer. A spectrometer is preferably combination of an optical element decomposing the light spatially into its spectral components and a plurality of detector elements, each arranged for converting the energy of one optical component collected over a certain period of time into an electric signal. A CCD sensor array is preferred as plurality of detector elements due to its costs advantages. In case of SS-OCT, the detector can be any device generating a signal depending on the incoming light, in particular detectors converting the energy of the light collected over a certain period of time, called integration time, into an electric signal.

In apparatus according to the invention, the reference arm comprises fiber optics as such optical elements allow for a particularly compact design. The fiber optics comprises at least one optical fiber having a fiber core, a fiber cladding and a fiber tip. The fiber tip defines the end of the optical fiber where the light is emitted from (output fiber tip) and/or coupled into the optical fiber (input fiber tip). The reference arm further comprises bulk optics comprising bulk optic components so as to facilitate operations on the reference light. The bulk optic components are arranged for collecting light exiting from an output fiber tip of said fiber optics and redirecting it to an input fiber tip of said fiber optics. The output fiber tip and the input fiber tip are preferably the same fiber tip to allow for a simple interferometer design, but separate input and output fiber tips are conceivable.

The intensity adjustment device facilitates the intentional misalignment of the reference light. It may comprise one or more optical elements which may change the geometric configuration of the light beam. The adjustment signal may be electric or optical. It is preferably electric so no further conversion for processing in a CPU or other electronic controlling device is needed. The adjustment signal may, however, not be manual, i.e. manual adjustment is not included in the scope of the invention, as the invention aims at allowing automatic regulation of the reference light.

Advantageously, the intensity adjustment device is arranged to modify the alignment of the light travelling through the reference arm by changing the alignment of the light redirected by the bulk optics with respect to the fiber core of said input fiber tip. This allows for a remarkably simple design as only the coupling of the reference light into the fiber is modified between practically perfect and imperfect modes. In this simple way, reproducible changes of alignments are achieved.

Advantageously, the apparatus according to the invention comprises an exposure control unit arranged for adjusting the integration time of the detection device, which exposure control unit is further arranged for generating said adjustment signal in dependence of said integration time. This facilitates automatic adjustment of the intensity of the returning reference light in dependence to the integration time. The exposure control unit preferably comprises a memory unit in which data representing a function of adjustment signals as a function of integration time may be stored. This allows for a fast adjustment of the reference light intensity in case of a change of integration time. The exposure control unit may be equipped with an automatic calibration function to generate the data to be stored in the memory unit. This function may operate in that way that the pivot angle is changed gradually within a predetermined range of angles and the signal strength received at the detection device is recorded, while the sample light intensity is either zero or constant.

Advantageously, the intensity adjustment device comprises a pivotable mirror, the pivot angle of which is adjustable in response to said adjustment signal. This allows for a simple design of an apparatus according to the invention. For the same reason, said pivotable mirror is preferably a galvanometric mirror. Its pivot angle can be controlled very easily with standard equipment. The pivotable mirror is preferably located at the terminal point of the reference arm, so the light impinging on the mirror surface is reflected along exactly the same path which it came if the mirror surface is oriented perpendicularly to the axis of the impinging light, but takes another path if the mirror is pivoted. In this configuration, an adjustable misalignment can be achieved very easily.

The method according to the invention may be performed with the above described apparatus according to the invention including the described improvements.

An embodiment of the invention as an example will now be described in greater detail with reference to drawings, in which
- Fig. 1: is a block diagram of an SOCT apparatus according to the invention on which the method according to the invention can be performed;
- Fig. 2: is an enlarged view of a portion of the reference arm of Fig. 1 with pivoted mirror;
- Fig. 3: is an en face view of the fiber tip of the refer- ence arm of Fig. 1;
- Fig. 4: is the same view as Fig. 3 with a symbolized im- pinging reference light beam;
- Fig. 5: is the same view as Fig. 4 with the reference light beam being misaligned.

A superluminescent diode (SLD) 1 acts as light source and emits light into an optical fiber system 2. The light is guided to an optical coupler 3 acting as beam splitter, dividing the light into a reference light directed into a reference arm and sample light directed into a sample arm. In this example, the optical coupler 3 also acts as recombining device superimposing the light returning from the reference arm with the light returning from the sample arm 9.

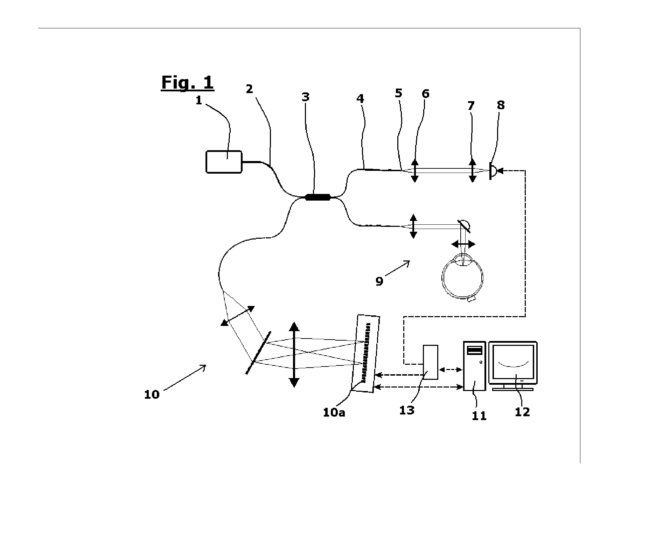

The first portion of the reference arm consists of an optical fiber 4 terminating at a fiber tip 5. At this fiber tip 5, the light exits the optical fiber system 2 to be collimated by a collimating lens 6. The collimated light beam passes a focusing lens 7 focusing the light onto the surface of a pivotable mirror 8. In Fig. 1, the mirror surface is aligned perpendicularly to the axis of the impinging light beam. It follows that the light beam returns along exactly the same geometric route as along which it has travelled towards the mirror 8. In this configuration, the alignment is practically perfect so that a maximum portion of light is coupled into the optical fiber system 2 at fiber tip 5 of the reference arm fiber 4.

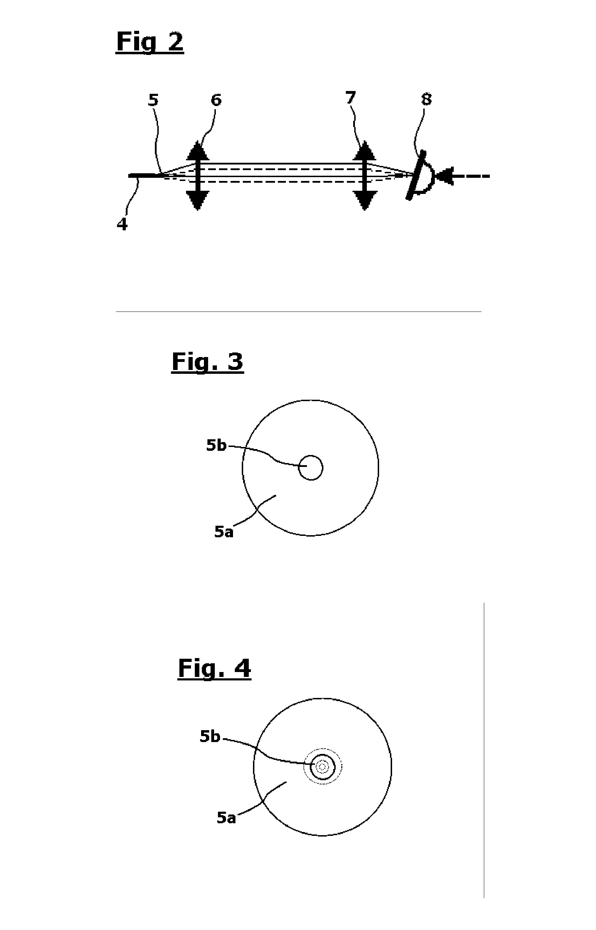

Fig. 3 shows in greater detail the front surface of the fiber tip 5 having a core 5a and a cladding 5b. If the reference light is practically perfectly aligned as shown in

Fig. 1, it impinges virtually centrally onto the core 5a as shown in Fig. 4, where the dotted circles symbolize the cross-section of the light beam with the highest intensity being localized in the center.

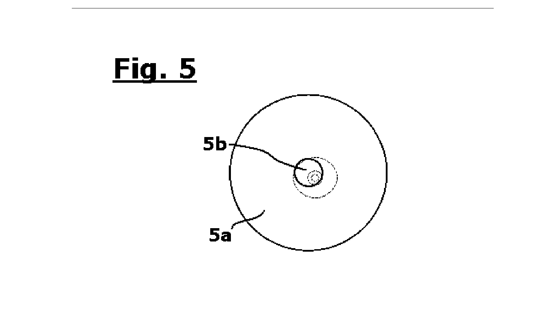

Fig. 2 shows a section of the reference arm including the pivotable mirror 8. Here, the mirror 8 is pivoted by a small angle. The angle in the drawing is exaggerated as compared with reality for illustrative purpose. The light travelling towards the mirror surface is symbolized by dotted lines, while the light reflected by the mirror 8 is represented by solid lines. The inclination of the mirror surface with respect to the incoming light causes the reflected light beam to be slightly misaligned so that it still reaches the fiber core 5a of the fiber tip 5, however not centrally so that only a smaller fraction of the reference light is coupled into the fiber system 2. This situation with imperfect alignment is visualized in Fig. 5. In this way, the reference light is attenuated in a remarkably simple and dispersion-free way.

As can be seen in Fig. 5, the misalignment is achieved by causing a portion of the light beam to impinge on the cladding 5b. Additionally or alternatively, in cases where optical fibers are used, the misalignment may be achieved by causing a portion of the light beam to impinge on the fiber core 5a, but in an angle outside the numerical aperture.

The returning reference light is superimposed by coupler 3 with the light returning from the sample arm 9. The sample arm 9 comprises the usual elements such as collimating lens and focusing lens for focusing the light onto the sample. The sample is not part of the apparatus of the invention. In Fig. 1, the sample is symbolized by an eye, but it may be any other structure of semi-transparent matter to be investigated with OCT. A pivotable mirror acting as scanning optics to allow for B scans is positioned between the collimating lens and the focusing lens.

The superimposed light is passed into a detection arm 10 comprising a spectrometer. Again, the spectrometer comprises the typical components as a collimating lens, a diffraction grating for spatial decomposition of the light, a focusing lens and a CCD sensor array 10a. The electric signals generated by the sensor array are read out be a frame grabber card (not shown) of a computer 11 which evaluates the signal and shows the calculated image on a display 12.

The sensor array 10a is further in communication with an exposure control unit 13. The exposure control unit 13 adjusts the integration time of the CCD sensor array. At the same time, it adjusts the pivoting angle of the pivoting mirror 8 in the reference arm. To this end, it is in communication with the pivotable mirror 8 so it can send an adjustment signal in response of which the pivot angle is changed. This is illustrated in Fig. 1 by a dotted arrow connecting control unit 13 to the pivotable mirror 8. In case of a decay of SNR, which may be signalled to the control unit 13 from the computer 11, the control unit 13 adjusts a longer integration time at the sensor array 10a and, simultaneously, pivots the mirror 8 in order to prevent the light energy at the sensor array 10a to surpass saturation level. The control unit 13 comprises a memory unit (not shown) in which the pivot angles are stored as a function of integration time. These data may be generated by calibration. The control unit 13 may be integrated into the computer 11 either as a hardware or software component or a combination thereof.

## Claims

1. Apparatus for Fourier domain optical coherence tomography comprising a light source (1), a reference arm, a sample arm (9), a recombining device (3) arranged for superimposing light returning from the reference arm with light returning from the sample arm (9) , a detection device (10a), whereas the reference arm comprises
fiber optics comprising at least one optical fiber (4) having a fiber core (5a), a fiber cladding (5b) and a fiber tip (5) , and whereas the reference arm further comprises bulk optics comprising bulk optic components (6, 7, 8) arranged for collecting light exiting from an output fiber tip (5) of said fiber optics and redirecting it to an input fiber tip (5) of said fiber optics, **characterized in that** the reference arm further comprises an intensity adjustment device arranged to modify the alignment of the light travelling through the reference arm in response to an adjustment signal.

2. Apparatus for Fourier domain optical coherence tomography according to claim 1, **characterized in that** said intensity adjustment device is arranged to modify the alignment of the light travelling through the reference arm by changing the alignment of the light redirected by the bulk optics with respect to the fiber core (5a) of said input fiber tip (5).

3. Apparatus for Fourier domain optical coherence tomography according to claim 1 or 2, **characterized in that** the apparatus further comprises an exposure control unit (13) arranged for adjusting the integration time of the detection device (10a), which exposure control unit (13) is further arranged for generating said adjustment signal in dependence of said integration time.

4. Apparatus for Fourier domain optical coherence tomography according to claim 3 or 2, **characterized in that** said exposure control unit (13) comprises a memory unit in which data representing a function of adjustment signals as a function of integration time may be stored.

5. Apparatus for Fourier domain optical coherence tomography according to any of the preceding claims, **characterized in that** said intensity adjustment device comprises a pivotable mirror (8), the pivot angle of which is adjustable in response to said adjustment signal.

6. Apparatus for Fourier domain optical coherence tomography according to claim 4, **characterized in that** said pivotable mirror (8) is a galvanometric mirror.

7. Method of performing Fourier domain optical coherence tomography comprising the steps of
- directing light from a light source (1) through a reference arm and a sample arm (9),
- superimposing light returning from the reference arm with light returning from the sample arm (9),
- analyzing the superimposed light detected over a certain period of time,
**characterized by**
- adjusting the alignment of the light travelling along the reference arm in dependence of said certain period of time.
